# EUROPEAN PATENT APPLICATION

(11) **EP 2 156 834 A1**
(43) Date of publication of application: **24.02.2010**
(21) Application number: 08425559.5
(22) Date of filing: 08.08.2008
(51) Int. Cl.: A61K 31/44, A61P 27/00, A61P 27/06

(54) **Ophthalmic pharmaceutical compositions comprising Sorafenib for the treatment of neoangiogenic pathologies of the eye**

(71) Applicant: S.I.F.I - SOCIETÀ INDUSTRIA FARMACEUTICA ITALIANA - S.P.A., Frazione Lavinaio 95020 Aci S. Antonio (IT)
(72) Inventor: Mazzone, Maria, Grazia, 95024 Acireale (IT); Papa, Vincenzo, 95027 San Gregorio Di Catania (IT); Giuliano, Francesco, 95037 S. Giovanni La Punta (IT); Marrano, Manuela, 95029 Viagrande (IT)
(74) Representative: Gervasi, Gemma

(57) **Abstract**

The present invention relates to ophthalmic pharmaceutical compositions comprising Sorafenib, its derivatives or active metabolites, for the treatment and prevention of ocular neoangiogenic pathologies.

## Description

### Field of the invention

The present invention relates to ophthalmic pharmaceutical compositions based on Sorafenib or its derivatives or active metabolites, associations or therapeutic combinations, suitable for the treatment or prevention of ocular neoangiogenic pathologies, in particular of the posterior chamber of the eye and the retina where there is neovessel formation, such as retinopathy of prematurity (ROP), choroidal neovascularization (CNV), age related macular degeneration (AMD), macular oedema, neovascular glaucoma and diabetic retinopathy (DR).

### State of the art

The proliferation of new blood vessels from pre-existing ones (angiogenesis or neoangiogenesis) is a necessary process for physiological tissue growth. In adults, however, this occurs only under specific conditions such as, in the female reproductive system, during ovulation and pregnancy, or during tissue repair (for example in wound repair). In such cases, the timing and extent of the phenomenon are generally controlled (Klagsbrun et al., Ann. Rev. Physiol., 1991, 53:217-239; Dorrell M et al., Survey of Ophthalmology, 2007, Vol.52, S1, s3-s19). In contrast, uncontrolled neoangiogenesis is implicated in the etiology of various pathologies such as: solid tumours, rheumatoid arthritis, psoriasis and, in the eye, corneal neovascularization, age related macular degeneration (AMD), macular oedema, retinopathy of prematurity (ROP), choroidal neovascularization (CNV), diabetic retinopathy (DR) (Folkman et al., J. Biol. Chem. 267:10931-10934 (1992); Klagsbrun et al., Ann. Rev. Physiol. 53:217-239 (1991); Dorrell M. et al., Survey of Ophthalmology, 2007, Vol.52, S1, s3-s19) and neovascular glaucoma.

Ferrara demonstrated the role of VEGF (Vascular Endothelial Growth Factor) in regulating physiological and pathological angiogenesis (Ferrara N. et al., Endocr. Rev., 18:4-25, 1997).

It has also been observed that high levels of VEGF in ocular tissues are correlated with proliferation of neovessels in diabetic patients or patients affected by other ischemic pathologies (Aiello L.P. et al., N. Engl. J. Med., 331:1480-1487, 1994), and that VEGF is localized in the choroidal neovascular membrane of patients affected by AMD (Lopez et al., Invest. Ophthalmol. Vis. Sci., 37:855-868, 1996). VEGF is a pro-angiogenic factor, different isoforms of which exist, acting in a specific manner by interacting with receptors of the VEGFR family coupled with tyrosine kinase (TK). Its principal effect is to induce extravasation of plasma proteins which results in the formation of an extravascular fibrin gel, being a substrate for the growth of neovessels. VEGF also induces the fenestration of endothelial cells (Ferrara N. et al., Endocr. Rev., 18:4-25, 1997).

The effects of VEGF isoforms on the vascular endothelium are shown schematically in the following tables 1, 2 and 3, taken from those scientific publications cited below them.

**Table 1**

| Ligand | Isoforms | Receptor | Biological activity |
|---|---|---|---|
| VEGF | VEGF-A₁₂₁ | VEGFR-1 and R-2; | Vasculogenesis, angiogenesis, |
| (VEGF-A) | VEGF-A₁₆₅ | VEGF₁₆₅ binds to | vascular homeostasis, vascular |
| | VEGF-A₁₈₉ | neuropilin-1 and -2; | permeability, recruitment of bone |
| | VEGF-A₂₀₆ | VEGF₁₆₅ binds to | marrow-derived cells |
| | (VEGF-A₁₃₈/₁₄₅/₁₆₂/_{165b}) | neuropilin-1 | |
| PIGF | PIGF₁₃₁ (PIGF-1) | VEGFR-1; | Angiogenesis, monocyte migration, |
| | PIGF₁₅₂ (PIGF-2) | PIGF₁₅₂ binds to | up-regulation of VEGF-A, recruitment |
| | PIGF₂₀₃ (PIGF-3) | neuropilin-1 and -2 | of bone marrow-derived cells |
| VEGF-B | VEGF-B₁₆₇ | VEGFR-1 and | Angiogenesis, recruitment of bone |
| | VEGF-B₁₈₆ | neuropilin-1 | marrow-derived cells |
| VEGF-C (VEGF-2) | | VEGFR-2 and -3 and neuropilin-2 | Development of lymphatic system and lymphangiogenesis, angiogenesis, |
| VEGF-D | | VEGFR-2 and -3 and neuropilin-2 | Lymphangiogenesis and angiogenesis |
| VEGF-E | | VEGFR-1 and neuropilin-1 | Angiogenesis, |
| VEGF-F | | VEGFR-2 | Angiogenesis, and vascular permeability |

The effects of VEGF (table modified from Ylä-Herttuala et al., JACC, 2007 (49):1015-1026)

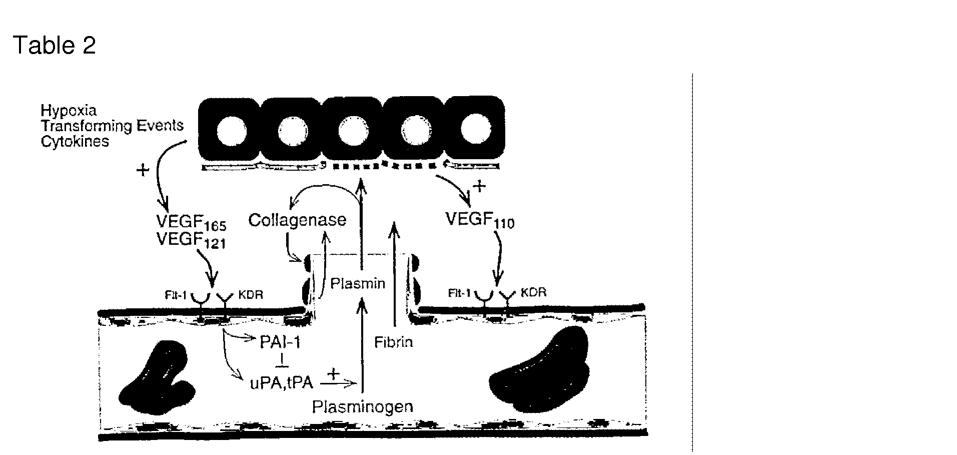

Schematic representation of the effects of VEGF on vascular endothelium (from Ferrara et al. Endocr. Rev., 18:4-25, 1997)

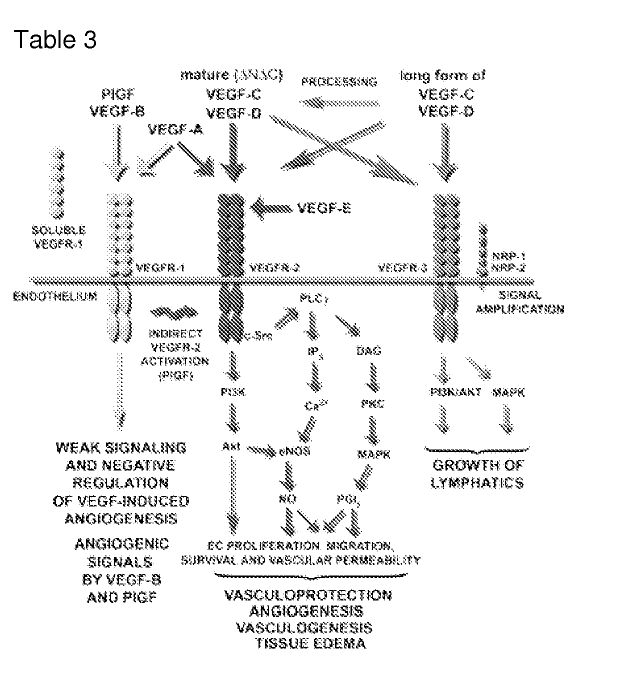

The effects of VEGF on vascular endothelium (from Ylä-Herttuala JACC 2007, 49, 1015-1026)

High levels of VEGF have been determined in the ocular tissues of patients affected by degenerative eye diseases associated with retinal ischemia such as: AMD, retinopathy of prematurity (ROP), diabetic retinopathy, CNV and neovascular glaucoma. In this respect, ischemia is considered to be one of the factors which induces up-regulation of VEGF (Ferrara N. et al., Endocr. Rev., 18:4-25, 1997; Aiello L.P. et al., N. Engl. J. Med., 331:1480-1487, 1994).

AMD is an age-related degenerative pathology of the retina resulting in a severe and irreversible reduction in visual acuity. The causes leading to the onset of AMD are not yet well known, but are thought to include the following: family history, smoking, diabetes, alcohol consumption and excessive exposure to the sun (D'Amico, N. Engl. J. Med., 331:95-106, 1994; Christensen et al., JAMA, 276:1147-1151, 1996).

AMD is characterized by abnormal neovascularization of the retina, iris and choroid (CNV) - with consequent formation of dysfunctional neovessels linked to possible leakages or haemorrhages - with possible associated retinal oedema, vitreoretinal haemorrhage or retinal detachment followed by a decline in visual acuity (VA).

CNV is characterized by various components: pre-existing neovascularization, further neovascularization and inflammation. The ideal therapy should therefore act in a concerted manner on all these components. Unfortunately currently available therapies act instead on the individual components of CNV and are not therefore sufficient *per* se to resolve the pathology, as can be seen in Table 4 which is presented for further clarity.

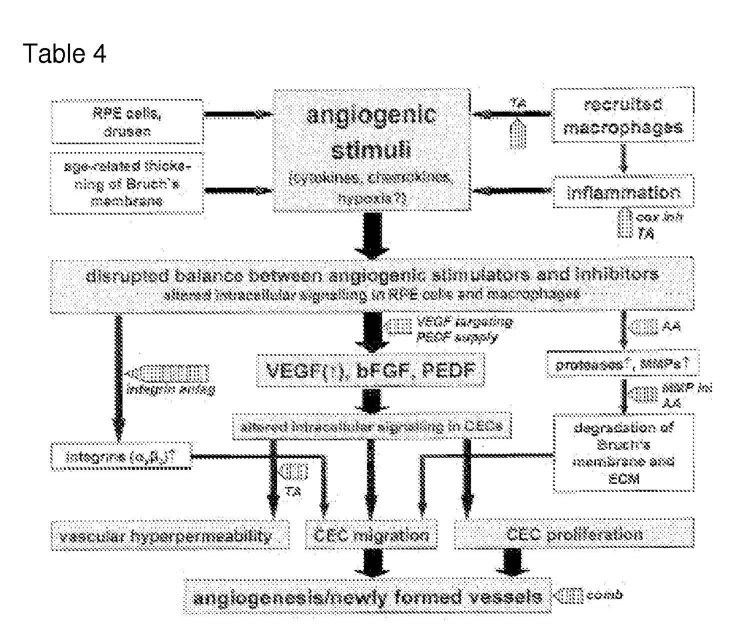

Angiogenic pathway (from Current Pharmaceutical Design, 2006, Vol. 12, 2645-2660)

CNV is, in fact, currently treated with a combined PDT therapy (photodynamic therapy with verteporfin, Visudyne®, Novartis) + anti-VEGF or PDT + steroids or angiostatic cortisenes. This therapeutic combination is carried out because PDT only acts on neovessels already existing at the start of therapy, by stabilizing the neovascular lesion and slowing down - without however halting - visual acuity decline in patients affected by CNV (Kaiser, Retina Today, May/June 2007). Etiopathological studies on CNV allowed to identify VEGF as one of the main factors involved in angiogenesis associated with retinal pathologies, being also involved, together with angiopoietin, TGF-α , TGF-β and other growth factors, in the development of tumours (Ferrara, Laboratory Investigation, 2007, 87, 227-230). In this respect, VEGF has pro-angiogenic effects such as vasodilation, increased vascular permeability and release of proteolytic enzymes, with consequent tissue remodelling. Studies carried out on anti-VEGFs (originally developed for oncological therapy) for CNV treatment, have led to the use of pegaptanib (Macugen^{®}, Pfizer), and ranibizumab (Lucentis^{®}, Genentech) in clinical therapy for CNV (Fig. 5.). In addition, bevacizumab (Avastin^{®}, Genentech), an anti-VEGF approved for breast, lung and rectal colon carcinoma therapy, is currently used "off label" in AMD therapy.

Anti-VEGFs act in a complementary manner to PDT as they inhibit progression of neovascularization, but do not act on pre-existing CNV: hence, for this reason, they are generally combined with PDT in clinical therapy.

In contrast to PDT, anti-VEGF therapy actually improves visual acuity (VA) in patients, but this effect is achieved only with frequent administrations (generally monthly) for an extended time period (Lee, American Academy of Ophthalmology 2007 Annual Meeting, Scientific Paper PA 060 presented Nov. 12, 2007). Steroids and angiostatic cortisenes are associated with PDT as they act on the inflammatory component of the pathology and on neovessel formation.

Diabetic retinopathy is a complication of diabetes which many diabetic patients develop over an approximately 20 years period. In this pathology, a number of phases can be recognized: 1. the precocious phase, i.e. the first phase of the disease, in which the retinal arteries weaken thus giving rise to leakage, microhaemorrhages and retinal oedema and hence to visual acuity decline; 2. the proliferative phase, in which the retina becomes ischemic and neovessels form in order to maintain adequate tissue oxygenation; 3. the late phase, characterized by further neovessel formation which can lead to other complications arising such as glaucoma or retinal detachment.

Therapy for diabetic retinopathy depends on the phase of disease progression of the patient. Neovessel formation is normally treated with PRP (Pan Retinal Photocoagulation) whereby the surgeon destroys by laser that part of the retina having become ischemic, in order to halt progression of the pathology. This technique however cannot be undertaken outside the macula and results in some blind areas in the peripheral part of the patient's field of view.

Even in the case of haemorrhagic type complications of the vitreous or retinal detachment, surgery is carried out: in the first case by a vitrectomy and in the second case by surgically reattaching the retina at its natural position.

Specific pharmacological therapies for the treatment of diabetic retinopathy do not exist up to now.

Macular oedema can be a complication of several ocular pathologies - such as AMD, diabetic retinopathy, severe uveitis and retinitis pigmentosa - or can be caused by surgical procedures to the eye (CMO or Cystoid Macular Oedema, caused for example by vitrectomy or cataract surgery) and is one of the most serious causes of blindness in industrialized Countries (Campochiaro P.A. et al., BJO 2000, 84:542-545).

Macular oedema is due to the extravasation of fluids from macular vessels, and when the fovea is involved, causes a decline in visual acuity due to dysfunction of retinal neurons (Campochiaro P.A. et al., BJO 2000, 84:542-545). Under physiological conditions, this extravasation is prevented by the functionality of blood-retinal barrier (BRB), but BRB dysfunctions lead to macular leakage with consequent formation of oedema.

As the specific etiopathogenic mechanisms of macular oedema are unknown, there are currently no specific therapies for this pathology. Often, when macular oedema is associated with diabetic retinopathy, anti-inflammatories are used and it has been observed that, when macular oedema follows cataract surgery, therapy with Non-Steroidal Anti-Inflammatory Drugs (NSAIDs) helps in preventing macular oedema and can improve visual acuity in individuals affected by macular oedema for over 6 months.

Although corticosteroids have proved to be not effective *versus* placebo in one clinical study, they are nevertheless often used in the treatment of macular oedema, whereas, in some types of macular oedema, certain carbonic anhydrase inhibitors (CAI), usually used in glaucoma therapy, have proved to be effective (Cox S.N. et al., Arch. Ophthalmol., 1988, 106:1190-1195; Fishman et al., BJO, 2007, 91:345-348). However many patients affected by macular oedema do not benefit by the currently available therapies, therefore making necessary to find new treatments for this pathology (Campochiaro, P.A. et al., BJO 2000, 84:542-545).

Neovascular glaucoma can derive from retinal ischemia consequent to diabetic retinopathy, retinal detachment or ocular ischemic syndrome. As in case of diabetic retinopathy, it is treated with PRP (Pan Retinal Photocoagulation) combined with surgery or drugs to reduce intraocular pressure.

However, a limiting factor in treating the mentioned pathologies is also the cost of those drugs identified as effective, although always partially and unsatisfactorily also due to the onset of side effects.

An idea of the costs can be deduced from Table 5, taken from the cited bibliographic source, which gives the clinical protocol of certain drugs and the relative annual cost of the therapy.

**Table 5**

| Antiangiogenic treatments, relative clinical protocol and annual cost of therapy | | | |
|---|---|---|---|
| Product | Price/unit (euro) | Units/year | Annual cost of therapy (euro) |
| Ranibizumab (Lucentis) 0.3 ml vial, 10 mg | 2,019 | 5 | 10,097 |
| Pegaptanib (Macugen) 0.3 mg vial | 1,534 | 9 | 13,806 |
| Veteporfin (Visudyne) 10 ml vial,15 mg | 1,662 | 3 | 4,986 |

Scheme of clinical protocols and relative annual costs for AMD therapy (single product therapy)
Source: www.pharmamarketing.it

### Summary of the Invention

The technical problem underlaying the present invention, as shown by the briefly summarized state of the art, is therefore to provide a new medicament for the prevention and therapy of neovascular pathologies of the eye, in particular of the posterior chamber of the eye and retina, which is as effective as the currently available anti-VEGF therapies (Macugen®, Lucentis®), but economically advantageous compared therewith and without the side effects of other more economical therapies currently used "off label" for the same pathologies, such as those caused by corticosteroids (glaucoma, cataracts).

### Detailed description of the invention

The Applicant has now surprisingly found that ophthalmic compositions comprising Sorafenib (4-[4-[[4-chloro-3-(trifluoromethyl)-phenyl]-carbamoylamino]-phenoxy]-N-methyl-pyridine-2-carboxamide, (BAY 43-9006), having formula 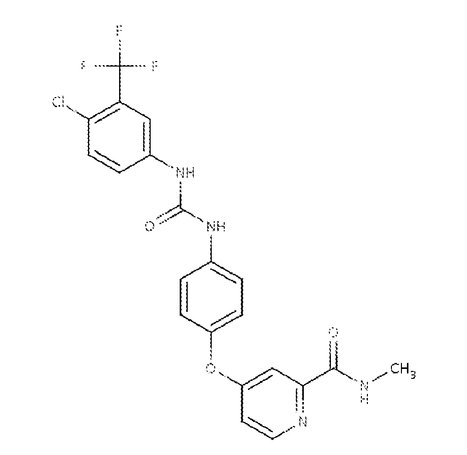 and its derivatives or active metabolites are capable to significantly reduce the incidence of retinal neoangiogenesis.

Sorafenib, further to a surprising antiangiogenic activity, has shown excellent chemical stability and unexpected therapeutic versatility compared to peptide, nucleotide and antibody type drugs.

At the same time, the present invention also relates to the use of Sorafenib, its derivatives or active metabolites, optionally in therapeutic association or combination with other active principles or other therapies of possible use, for the treatment of pathologies such as: CNV, AMD, ROP, macular oedema, neovascular glaucoma, and diabetic retinopathy. Usable drugs in therapeutic association or combination with Sorafenib, its derivatives or active metabolites are for example: monoclonal antibodies able to interfere with the neoangiogenesis pathway (e.g. ranibizumab), aptamers able to interfere with the neoangiogenesis pathway (e.g. pegaptanib), siRNA, VDA (vascular disrupting agent), angiostatic cortisenes, non-steroidal anti-inflammatory drugs (NSAIDs), corticosteroids, albendazole, mebendazole, CAI (carbonic anhydrase inhibitors), cannabinoids, COX-2 selective inhibitors, inhibitors of iNOS and molecules that, though not falling within these categories, act with one of the following action mechanisms: 1) anti-inflammatory; as interfering with inflammatory mediators such as: histamine, eicosanoids (prostanoids, leukotrienes), PAF (platelet activating factor), bradykinin, nitric oxide, neuropeptides (neurokinin A, substance P, calcitonin generated peptide - CGRP) and cytokines (interleukins, chemokines, interferons, growth factors - GF, tumour necrosis factors - TNF, macrophage colony stimulating factors (M-CSF) or granulocyte colony stimulating factors (G-CSF); 2) immunosuppressive: methotrexate, mesalazine and derivatives (sulfasalazine, balsalazine, ...), cyclosporine, azathioprine, leflunomide, tacrolimus and the like (pimecrolimus, sirolimus, gusperimus, everolimus), mycophenolate mofetil, thalidomide, lenalidomide.

The present invention also includes therapeutic associations or combinations of Sorafenib, its derivatives or active metabolites, with antimicrobial agents such as beta-lactamines (penicillins, cephalosporins), macrolides, tetracyclines, fluoroquinolones and natural polypeptides and fragments thereof having antibacterial action. These latter comprise for example: lysozyme, lactoferrin, melanocortin, thrombocidin, and other antimicrobial peptides which can be divided, by composition and by secondary structure, into three main groups: 1) peptides containing cysteine: a) peptides containing several disulphide bridges which adopt an antiparallel b sheet structure (defensin, tachiplesin); b) peptides with loop structure containing a single disulphide bridge (bactenecin, brevinin, esculentin); 2) peptides containing a high percentage of specific amino acids (PR-39, apidaecin and bovine peptides rich in proline and arginine Bac5 and Bac7); 3) linear peptides structured in an α-helix shape in a hydrophobic environment but adopting a random conformation in solution; this is the most numerous and well-studied group, which most of the classical antimicrobial peptides belong to and of which a great many examples are known (cecropin, melittin, magainin, dermaseptin, temporin, bombinin).

A further aspect of the invention is also the therapeutic combination of Sorafenib with other therapies used in clinical practice, such as PDT or surgical techniques. Sorafenib (BAY 43-9006, Bayer) is a diaryl urea acting on multiple targets (VEGF, PDGF, EGF; in this respect it is defined as a multikinase inhibitor) with prevalent anti-VEGF activity and possessing antiangiogenic action demonstrated in a tumour environment (EP 1140840B1, Bayer) and is the first antitumour drug approved in Europe for the treatment of hepatocellular carcinoma (Nexavar® tablets).

Sorafenib is produced by chemical synthesis, with a less expensive industrial process than those needed for producing anti-VEGFs currently used in retinal neovascular disease therapy, such as pegaptanib and ranibizumab. Sorafenib inhibits VEGF endothelial receptor tyrosine kinases (it is a TKI, i.e. Tyrosine Kinase Inhibitor).

A Bayer's patent application (PCT/EP2006/001191) also confirmed the therapeutic action of Sorafenib in the treatment of inflammatory type diseases of the skin, eyes and ears, possibly in combination with other active principles. Said patent application considers in particular inflammatory eye diseases without an angiogenic component (retinitis, uveitis and keratitis).

It should be noted that both angiogenesis and inflammation can appear independently of one another, although the simultaneous presence of the two components gives rise to a more serious pathology. In this respect, the simple use of anti-inflammatories is unable to resolve angiogenesis, nor those ocular pathologies related thereto, as described by Castro M. R. et al. (Experimental Eye Research, 2004, 79: 275-285) and also in the introduction to the present patent application.

Taking into consideration the structural complexity of the components of the posterior chamber of the eye and retina (such as vitreous body, RPE -retinal pigmented epithelium - and photoreceptors), the possible use of active principles that may be employed for treating eye surface diseases is not obvious to the expert of the art, because active principles and excipients that are, respectively, effective and well tolerated at the eye surface, can result to be ineffective or toxic for the posterior chamber of the eye and the retina.

Some authors report a few cases where the systemic (oral) use of Sorafenib administered for oncological therapy in patients also affected by choroidal neovascularization, and simultaneously treated with other intravitreal anti-VEGF drugs (Kernt. M et al, Acta Ophthalmologica 2008, 86: 456-458), or where Sorafenib was administered in an attempt to increase the time interval between intravitreal administrations of other anti-VEGFs for retinal neovascularization (Diago T. et al, Mayo Clin Proc, 2008, 83 (2): 231-234), has led to an improvement in VA.

In view of the simultaneous administration of several drugs, these results are difficult to interpret. Moreover, considering the complex mechanism of action of Sorafenib, it is preferable to avoid the systemic route for therapy of ocular pathologies, as the active principle would interfere with various physiological pathways, thus giving rise to side effects (the most common being: nausea, weakness or tiredness, painful mouth and abdomen, headache, painful bones, hair loss, redness, redness or painful palms of the hands or soles of the feet - hand/foot syndrome - itching or skin blemishes, vomiting, bleeding of the intestinal walls and respiratory tract, cerebral haemorrhage, hypertension) which, in ocular pathologies, would be considered more severe than in oncology therapy.

In addition, there are many interactions of Sorafenib with other drugs which would be related to their systemic administration (EPAR Nexavar®).

The therapeutic index of Sorafenib for the disease therapy of the invention can be increased by the use of local administration to the eye: the pharmacological effect can hence be obtained while limiting the onset of undesired effects due to the reduced systemic absorption. Furthermore, local administration enables lower doses to be used than are needed to give the same effect by systemic administration.

Sorafenib, its derivatives and its active metabolites are lipohilic molecules, practically insoluble in water, thereby should be suitably formulated. Various types of compositions can be considered for the administration of an active principle of this type, such as the following non-limiting examples: suspensions, ointments, solid inserts, gels, emulsions, oily solutions, solutions obtained by means of cyclodextrins, liposomes, nanosystems or depots.

The present invention also includes soluble derivatives of Sorafenib and its active metabolites and their ophthalmic compositions, associations or therapeutic combinations.

A further aspect of the invention is the ophthalmic use of the above disclosed compositions for the treatment or prevention of pathologies of the posterior chamber of the eye and retina. Said compositions can be compatible with topical, subconjunctival, intravitreal, subretinal, periocular, retrobulbar or juxtascleral ocular administration. The invention also includes the use of the described compositions for the treatment and prevention of pathologies of the posterior chamber of the eye and the retina, comprising the administration, to a patient requiring it, of a therapeutically effective amount of Sorafenib in the compositions as above disclosed. Among the pathologies of the posterior chamber of the eye and the retina effectively treated for the purposes of the invention, the following can be cited: age-related macular degeneration (AMD), choroidal neovascularization (CNV), proliferative vitreoretinopathy, retinopathy of prematurity (ROP), retinal vascular diseases, macular oedema, diabetic retinopathy, neovascular glaucoma and implications of systemic pathologies for the retina. The treatment with Sorafenib can be optionally carried out in therapeutic association or in combination with other therapies for the treatment of the same pathologies, as previously described.

Said compositions form the subject of the invention.

### EXPERIMENTAL PART

### Sorafenib efficacy tests in a rat model of choroidal neovascularization (CNV)

Experiments were carried out on Brown Norway rats having average weight of 180-220 g, which were randomized, divided into treatment groups and anaesthetized by intraperitoneal injections of ketamine and xylazine.

After anaesthesia, tropicamide was administered to dilate the pupils. In each eye a few laser lesions were made in the space between large calibre retinal vessels using an argon photocoagulation laser (wavelength: 485-514 nm; power: 90 mW; duration: 0.1 sec.; lesion size: 100 µm).

The eyes subjected to photocoagulation were treated - based on the group they belonged to - with 5µl of the relative treatment, injected into the vitreous by means of a Hamilton syringe provided with a 30-Gauge needle.

The efficacy of the compositions containing Sorafenib was determined and compared with the control group treated with the vehicle.

Two weeks after the treatment, 100 µl of a fluorescein sodium solution were injected into the caudal vein of each animal in order to carry out the angiography. The incidence of choroidal neovascularization (CNV) was determined on the basis of the angiography and the lesions caused by the laser were classified as positive or negative on the basis of the presence or absence of hyperfluorescence of the lesions themselves.

### Results

The treatment with Sorafenib led to a significant reduction in the incidence of CNV compared to the control.

### Conclusions

The experimental data show that Sorafenib is effective in the experimental model of CNV, used for establishing the efficacy of drugs in retinal neovascular disease therapy.

## Claims

1. Ophthalmic pharmaceutical compositions comprising Sorafenib, its derivatives or active metabolites, for the therapy and prevention of ocular neoangiogenic pathologies.

2. Ophthalmic pharmaceutical compositions comprising Sorafenib, its derivatives or active metabolites, for the therapy and prevention of ocular neoangiogenic pathologies in the posterior part of the eye.

3. Ophthalmic pharmaceutical compositions according to claim 2, comprising Sorafenib, its derivatives or active metabolites, for the therapy and prevention of neoangiogenic pathologies of the retina.

4. Pharmaceutical compositions according to claim 1 further comprising VDAs, corticosteroids, angiostatic cortisenes, albendazole, mebendazole, carbonic anhydrase inhibitors (CAI), cannabinoids, non-steroidal anti-inflammatory drugs (NSAIDs), COX-2 selective inhibitors, inhibitors of iNOS, molecules that interfere with inflammatory mediators, molecules with immunosuppressive action, beta-lactamines (penicillins, cephalosporins), macrolides, tetracyclines, fluoroquinolones, natural polypeptides with antibacterial action and fragments thereof, aptamers able to interfere with the neoangiogenesis pathway, monoclonal antibodies able to interfere with the neoangiogenesis pathway, SiRNAs.

5. Pharmaceutical compositions according to claim 1 in the form of: emulsions, ointments, solid inserts, suspensions, gels, oily solutions, viscosized solutions, solutions obtained by means of cyclodextrins, liposomes, nanoparticles, micelles, depots.

6. Ophthalmic use of Sorafenib, its derivatives or active metabolites, for the production of a medicament for the therapy and prevention of ocular pathogens with a neoangiogenic component.

7. Use of Sorafenib, its derivatives or active metabolites according to claim 1 wherein the neoangiogenic pathology relates to the posterior part of the eye.

8. Use of Sorafenib, its derivatives or active metabolites according to claim 7 wherein the neoangiogenic pathology relates to the retina.

9. Use of Sorafenib, its derivatives or active metabolites according to claim 6 for the production of an ophthalmic medicament for the treatment of: proliferative vitreoretinopathies, ROP, retinal vascular pathologies, implications of systemic pathologies for the retina, AMD, diabetic retinopathy, macular oedema, neovascular glaucoma, CNV.

10. Use of Sorafenib, its derivatives or active metabolites for the production of medicaments suitable for ocular administration, according to claim 6 whereby the administration is: topical, intravitreal, subconjunctival, subretinal, periocular, by juxtascleral injection, by retrobulbar injection.

11. Use of the ophthalmic pharmaceutical compositions according to claim 1 suitable for the administration by ocular route, whereby the administration is: topical, intravitreal, subconjunctival, subretinal, periocular, by juxtascleral injection, by retrobulbar injection.

12. Use of the ophthalmic pharmaceutical compositions according to claim 1 in therapeutic combination with PDT.

13. Use of the ophthalmic pharmaceutical compositions according to claim 1 in therapeutic combination with: VDAs, corticosteroids, angiostatic cortisenes, albendazole, mebendazole, carbonic anhydrase inhibitors (CAI), cannabinoids, non-steroidal anti-inflammatory drugs (NSAIDs), COX-2 selective inhibitors, inhibitors of iNOS, molecules that interfere with inflammatory mediators, molecules with immunosuppressive action, beta-lactamines (penicillins, cephalosporins), macrolides, tetracyclines, fluoroquinolones, natural polypeptides with antibacterial action and fragments thereof, aptamers able to interfere with the neoangiogenesis pathway, monoclonal antibodies able to interfere with the neoangiogenesis pathway, siRNAs.
